(19) Europäisches Patentamt
European Patent Office
Office européen des brevets

(11) Veröffentlichungsnummer : **0 347 693 B1**

# (12) EUROPÄISCHE PATENTSCHRIFT

(45) Veröffentlichungstag der Patentschrift : **09.09.92 Patentblatt 92/37**

(51) Int. Cl.$^5$ : **C07C 33/20,** C07C 47/542, C07C 255/50, C07C 29/00, C07C 45/44, C07C 253/14

(21) Anmeldenummer : **89110561.1**

(22) Anmeldetag : **10.06.89**

(54) **Neue Benzonitrile, Benzaldehyde und Benzylalkohole sowie Verfahren zu ihrer Herstellung.**

(30) Priorität : **21.06.88 DE 3820896**

(43) Veröffentlichungstag der Anmeldung : **27.12.89 Patentblatt 89/52**

(45) Bekanntmachung des Hinweises auf die Patenterteilung : **09.09.92 Patentblatt 92/37**

(84) Benannte Vertragsstaaten : **AT BE CH DE ES FR GB IT LI NL**

(56) Entgegenhaltungen :
**EP-A- 0 276 766**
**DD-A- 151 742**
**DE-A- 2 550 261**
**DE-A- 3 118 682**
**GB-A- 2 162 846**

(73) Patentinhaber : **BASF Aktiengesellschaft**
**Carl-Bosch-Strasse 38**
**W-6700 Ludwigshafen (DE)**

(72) Erfinder : **Wolf, Bernd, Dr.**
**Eichenstrasse 11**
**W-6704 Mutterstadt (DE)**
Erfinder : **Theobal, Hans, Dr.**
**Queichstrasse 6**
**W-6703 Limburgerhof (DE)**
Erfinder : **Goetz, Norbert, Dr.**
**Schoefferstrasse 25**
**W-6520 Worms 1 (DE)**

EP 0 347 693 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Benzonitrile, Benzaldehyde und Benzylalkohole der allgemeinen Formel I

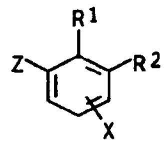

I,

in der
$R^1$ Methyl oder Ethyl,
$R^2$ Alkyl, Alkenyl, Cycloalkyl, Cycloalkenyl, Bicycloalkyl, Bicycloalkenyl oder $C_1$-$C_5$-alkylsubstituiertes Cycloalkyl, Cycloalkenyl, Bicycloalkyl oder Bicycloalkenyl,
X Wasserstoff, Chlor oder Fluor und
Z die Gruppe -CN, CHO oder

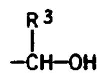

,

wobei $R^3$ für Wasserstoff, Cyano, $C_2$-$C_4$-Alkinyl, $C_2$-$C_4$-Alkenyl oder $C_1$-$C_4$-Alkyl steht,
bedeuten, mit der Maßgabe, daß $R^2$ nicht den Rest $-CH_2-CH=CH-B$ darstellt, wenn B für Wasserstoff, Alkyl oder Alkenyl steht und zugleich $R^1$ die Bedeutung Methyl und Z die Bedeutung

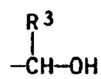

hat sowie mit der Maßgabe, daß $R^2$ nicht Methyl oder Ethyl bedeutet, wenn zugleich $R^1$ für Methyl und Z für

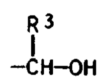

steht und ferner mit der Maßgabe, daß $R^2$ nicht Methyl bedeutet, wenn zugleich $R^1$ für Methyl oder Ethyl und Z für die Gruppe -CN oder -CHO steht, sowie Verfahren zu ihrer Herstellung.

2,3-Dimethylbenzylalkohol, 3-Ethyl-2-methylbenzylalkohol und 2,3-Dimethyl-α-methylbenzylalkohol sind z.B. in J. Chem. Soc., Perkin Trans. 1 (12), 3087-91 (1981); J. Chem. Soc., Perkin Trans. 1, (20), 2339-42 (1974); Helv. Chim. Acta, 60 (5), 1758-80 und Tetrahedron Lett. 22, 161-162 (1981) beschrieben.

Weiterhin sind aus Pestic. Sci. 17 (6), 691-700 (1986) einige ortho-Methylbenzylalkohole bekannt, die in meta-Position einen gegebenenfalls endständig substituierten Allylrest tragen.

2,3-Dimethylbenzaldehyd und 2-Ethyl-3-methylbenzaldehyd sind in J. Chem. Soc., Perkin Trans. 1, 3087-91 (1981), J. Org. Chem., 47, 1361-1364 (1982) und Indian J. Chem., Sect. B, 25B, 1112-17 (1986) beschrieben.

2,3-Dimethylbenzonitril, 2-Ethyl-3-methylbenzonitril und 4-Chlor-2,3-dimethylbenzonitril sind aus J. Chem. Soc., Perkin Trans. 2, 1003-10 (1983) Indian J. Chem. Sect. B, 25B, 1112-17 (1986) und J. Chem. Soc., 1964, 2258-61 bekannt.

Der Erfindung lag die Aufgabe zugrunde, neue Zwischenprodukte zur Herstellung von Schädlingsbekämpfungsmitteln, insbesondere von Benzylestern der Struktur IV

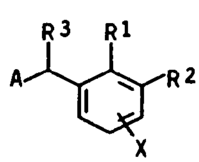

IV,

worin A den Carboxylatrest einer bei Pyrethroiden typischen Säurekomponente und die Reste $R^1$ bis $R^3$ und X die oben genannte Bedeutung haben, zur Verfügung zu stellen und Verfahren zur Herstellung dieser

Zwischenprodukte aufzufinden.

Es wurde nun gefunden, daß die eingangs definierten Verbindungen zur Herstellung solcher Benzylester IV, die eine gute insektizide und akarizide Wirksamkeit aufweisen und in der zeitgleichen deutschen Anmeldung P 38 20 895.4 EP-A-0347694 beschrieben sind, besonders geeignet sind.

Die Benzylester der Formel IV können durch Umsetzung der Säuren A-H, von denen einige typische Vertreter nachstehend aufgeführt sind, oder Derivaten dieser Säuren wie Säurechloriden, Säureanhydriden o.ä. mit Benzylalkoholen der allgemeinen Formel Ia oder Derivaten davon nach folgendem Reaktionsschema gewonnen werden.

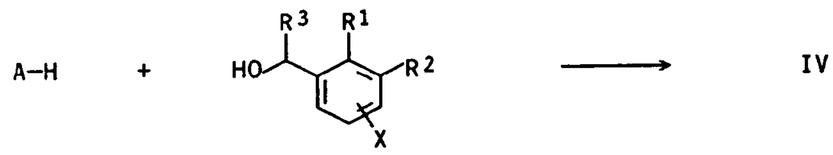


Die Reste $R^1$ bis $R^3$ und X haben die voranstehend genannte Bedeutung.

Typische Repräsentanten der Säuren der Formel A-H sind z.B.:

$A^1$-H: 3-(2′2′-Dimethylvinyl)-2,2-dimethylcyclopropan-2-carbonsäure
$A^2$-H: 3-(2′2′-Dichlorvinyl)-2,2-dimethylcyclopropan-1-carbonsäure
$A^3$-H: 3-(2′-Chlor-3′,3′,3′-trifluorprop-1′-enyl)-2,2-dimethylcyclopropan-1-carbonsäure
$A^4$-H: 3-(2′,2′-Dibromvinyl)-2,2-dimethylcyclopropan-1-carbonsäure
$A^5$-H: 3-(2,2′-Difluorvinyl)-2,2-dimethylcyclopropan-1-carbonsäure
$A^6$-H: 3-(2′-Fluor-3′,3′,3′-trifluorprop-1′-enyl)-2,2-dimethylcyclopropan-1-carbonsäure
$A^7$-H: 3-(2′,2′-Bistrifluormethyl-vinyl)-2,2-dimethylcyclopropan-1-carbonsäure
$A^8$-H: 2-(4′-Chlorphenyl)-3-methylbuttersäure
$A^9$-H: 2-(4′-Fluorphenyl)-3-methylbuttersäure
$A^{10}$-H: 2-(4′-Difluormethoxyphenyl)-3-methylbuttersäure
$A^{11}$-H: 3-(4′-tert.-Butylphenyl)-2,2-dimethylcyclopropan-1-carbonsäure
$A^{12}$-H: 2,2,3,3-Tetramethylcyclopropan-1-carbonsäure
$A^{13}$-H: 1-(4′-Chlorphenyl)-cyclopropan-1-carbonsäure
$A^{14}$-H: 1-(4′-Ethoxyphenyl)-2,2-dichlorcyclopropan-1-carbonsäure
$A^{15}$-H: 3-[2′-(4$H_2$-Chlorphenyl)-2′-chlorvinyl]-2,2-dimethylcyclopropan-1-carbonsäure
$A^{16}$-H: 3-(1′,3′-Butadienyl)-2,2-dimethyl-cyclopropan-1-carbonsäure
$A^{17}$-H: 3-(2′-Methyl-2′-methoxycarbonyl-vinyl)-2,2-dimethylcyclopropan-1-carbonsäure
$A^{18}$-H: 2-(2′-Chlor-4′-trifluormethyl-phenylamino)-3-methylbuttersäure
$A^{19}$-H: 2-(2′-Fluor-4′-trifluormethyl-phenylamino)-3-methylbuttersäure
$A^{20}$-H: 3-Methyl-2-(4′-trifluormethyl-phenylamino)-buttersäure
$A^{21}$-H: 2-Methyl-2-(pyrrol-1′-yl)-buttersäure
$A^{22}$-H: 3-Methyl-2-(3′-methylpyrrol-1′-yl)-buttersäure
$A^{23}$-H: 2-(3′,4′-Dimethylpyrrol-1′-yl)-methylbuttersäure
$A^{24}$-H: 2-(2′,5′-Dimethylpyrrol-1′-yl)-methylbuttersäure
$A^{25}$-H: 2-(Isoindolin-2-yl)-3-methylbuttersäure
$A^{26}$-H: 1,1-Dimethyl-2,2[H]indenspirocyclopropan-3-carbonsäure
$A^{27}$-H: 3-Cyclopentylidenmethyl-2,2-dimethylcyclopropan-1-carbonsäure
$A^{28}$-H: 3-(1′,2′-Dibrom-2′,2′-dichlorethyl)-2,2-dimethylcyclopran-1-carbonsäure
$A^{29}$-H: 3-Methyl-2-(pyrazol-1′-yl)-buttersäure
$A^{30}$-H: 3-Methyl-2-(imidazol-1′-yl)-buttersäure

Die Umsetzung kann in an sich bekannter Weise durch Zusatz von Katalysatoren wie Schwefelsäure, Halogenwasserstoff, Sulfonsäuren, sauren Ionenaustauschern beschleunigt und das Veresterungsgleichgewicht im gewünschten Sinne verschoben werden, indem man dem Reaktionsgemisch das Wasser oder den Ester IV entzieht, z.B. durch azeotrope Destillation oder durch Bindung des Wassers an Schwefel- oder Halogenwasserstoffsäure.

Ebensogut können die entsprechenden Säurechloride mit den Alkoholen der Formel Ia in Gegenwart eines Säureakzeptors umgesetzt werden (vgl. Houben-Weyl, Methoden der organ. Chemie, Band VIII, S. 541 ff, Georg-Thieme-Verlag, Stuttgart 1952).

Als Säurebindemittel eignen sich die üblichen basischen Mittel, insbesondere aliphatische, aromatische und heterocyclische Amine, z.B. Triethylamin, Dimethylamin, Piperidin, Dimethylanilin, Dimethylbenzylamin,

Pyridin und 2-Picolin.

Die Umsetzung kann in einem Lösungs- oder Verdünnungsmittel ausgeführt werden. Hierzu sind die angeführten Säureakzeptoren selbst oder beispielsweise folgende Lösungs- oder Verdünnungsmittel oder Gemische derselben geeignet:

Aliphatische und aromatische, gegebenenfalls chlorierte Kohlenwasserstoffe, wie Petrolether, Benzol, Toluol, Xylol, Benzin, Dichlormethan, Chloroform, Tetrachlormethan, 1,2-Dichlorethan, Chlorbenzol; Ether wie Diethyl- und Di-n-butylether, Methyl-tert.-butylether, Tetrahydrofuran, Dioxan; Ketone, beispielsweise Aceton, Methylethylketon, Methylisopropylketon; ferner Nitrile wie Acetonitril und Propionitril.

Üblicherweise setzt man die Ausgangsstoffe in stöchiometrischen Verhältnis ein. Ein Überschuß des einen oder anderen kann in Einzelfällen aber durchaus vorteilhaft sein.

Die Umsetzung verläuft gewöhnlich oberhalb von 0°C mit ausreichender Geschwindigkeit. Da sie meist unter Wärmeentwicklung verläuft, kann es von Vorteil sein, eine Kühlmöglichkeit vorzusehen.

In einigen Fällen ist es sinnvoll und vorteilhaft die Verbindungen der Formel Ia, in situ zu verestern, insbesondere dann, wenn $R^3$ in der allgemeinen Formel Ia die Bedeutung einer Cyanogruppe hat.

Die erfindungsgemäßen Ester können außerdem noch nach praktisch allen bekannten Verfahren der Estersynthese hergestellt werden, beispielsweise durch Umsetzung von entsprechenden Säureanhydriden mit den Alkoholen der Formel Ia, durch Umsetzung von entsprechenden Salzen mit Derivaten der Alkohole der Formel Ia oder durch Umesterung (vgl. Houben-Weyl a.a.O. S. 508-628).

In der vorliegenden Anmeldung haben, falls nicht anders angegeben, die folgenden Bezeichnungen die folgende Bedeutung:

"Alkyl" bezieht sich auf eine gerade oder verzweigte Alkylgruppe mit 1 bis 20 insbesondere 1 bis 12 Kohlenstoffatomen. Beispielsweise seien Methyl, Ethyl, Propyl, Isopropyl, n-Butyl, iso-Butyl, sec.-Butyl und tert.-Butyl genannt.

"Alkenyl" bedeutet eine gerade oder verzweigte, ethylenisch ungesättigte Kohlenwasserstoffgruppe mit 2 bis 20 Kohlenstoffatomen und 1 bis 10 ethylenischen Bindungen, insbesondere mit 2 bis 12 Kohlenstoffatomen und 1 bis 5 ethylenischen Bindungen. Beispielsweise seien Vinyl, Isopropenyl, 1-Butenyl, 1,5-Hexadienyl, 1-Methyl-propenyl und 1-Ethyl-vinyl genannt.

Die Bezeichnung "Cycloalkyl" bedeutet eine Cycloalkylgruppe mit 3 bis 8, insbesondere 3 bis 6 C-Atomen, im Ring wie Cyclopropyl, Cyclobutyl, Cyclopentyl oder Cyclohexyl. Diese Cycloalkylgruppe ist unsubstituiert oder substituiert durch ein oder mehrere, z.B. 1 bis 4 verzweigte oder unverzweigte $C_1$-$C_5$-Alkylreste wie Methyl, Ethyl, Propyl, Isopropyl, Butyl, sec-Butyl, tert-Butyl oder Pentyl. Beispielsweise seien 3,5-Diethylcyclohexyl und Tetramethylcyclopropyl genannt.

"Cycloalkenyl" bezieht sich auf eine Cycloalkenylgruppe mit 3 bis 8, insbesondere 3 bis 6 C-Atomen, im Ring Cyclopropenyl, Cyclobutenyl, Cyclopentenyl, Cyclopentadienyl oder Cyclohexadienyl. Diese Cycloalkenylgruppe ist unsubstituiert oder substituiert durch ein oder mehrere z.B. 1 bis 4 verzweigte oder unverzweigte $C_1$-$C_5$-Alkylreste wie Methyl, Ethyl, Propyl, Isopropyl, Butyl, sec-Butyl, tert-Butyl oder Pentyl. Beispielsweise seien 1-Cyclopentenyl, 1-Cyclohexenyl, 3,5-Dimethyl-1-cyclohexenyl und 1,3-Cyclohexadienyl genannt.

"Bicycloalkyl" bedeutet eine Bicycloalkylgruppe mit 5 bis 12, insbesondere 6 bis 8 Kohlenstoffatomen im Bicyclus wie z.B. 2-Norbornyl oder Bicyclo[4.1.0]hept-1-yl. Dieser Bicyclus ist unsubstituiert oder substituiert durch ein oder mehrere, z.B. 1 bis 2 verzweigte oder unverzweigte Alkylreste wie Methyl, Ethyl, Propyl, Isopropyl, Butyl, sec-Butyl oder Pentyl. Beispielsweise sei 2,6-Dimethyl-bicyclo[4.1.0]hept-1-yl genannt.

"Bicycloalkenyl" steht für eine ungesättigte Bicycloalkylgruppe mit 5 bis 12, insbesondere 6 bis 8 Kohlenstoffatomen im Bicyclus und einer oder mehreren z.B. 1 oder 2 ethylenischen Doppelbindungen, wie z.B. Norbornen-2-yl, Norbornadien-2-yl. Diese Bicycloalkenylgruppe ist unsubstituiert oder substituiert durch 1 oder mehrere, z.B. 1 bis 3 verzweigte oder unverzweigte $C_1$-$C_5$-Alkylreste wie Methyl, Ethyl, Propyl, Isopropyl, Butyl, sec-Butyl oder Pentyl. Beispielsweise sei 7,7-Dimethylbicyclo[4.1.0]hept-2-en-1-yl genannt.

$R^3$ steht für Wasserstoff, Cyano, $C_2$-$C_4$-Alkinyl wie Ethinyl, Propin-1-yl, Propin-2-yl, Propin-2-yl oder Butinyl, $C_2$-$C_4$-Alkenyl wie Vinyl, Allyl oder Butenyl, $C_1$-$C_4$-Alkyl wie Methyl, Ethyl, Propyl, Isopropyl oder Butyl.

Bevorzugt stehen in den Verbindungen I für

$R^1$ Methyl,

$R^2$ verzweigte Alkyl- oder Alkenylreste mit 3 bis 8 Kohlenstoffatomen wie Isopropyl, Isopropenyl, sec-Butyl, 1-Buten-2-yl, 1-Methyl-1-propenyl, 1,3-Butadienyl, iso-Pentyl, sec-Pentyl, 3-Penten-3-yl, 1-Penten-2-yl oder sec-Hexyl, wobei die drei erstgenannten Reste besonders bevorzugt sind; $C_3$-$C_8$-Cycloalkyl oder Cycloalkenyl, wie Cyclopentyl, 1-Cyclopentyl, Cyclohexyl, 1-Cyclohexenyl, Cyclopropyl; $C_6$-$C_8$-Bicycloalkyl-oder Bicycloalkenyl wie 2-Norbornyl, 2-Norbornen-2-yl oder 2,5-Norbornadien-2-yl,

$R^3$ Wasserstoff, Ethinyl oder Cyano und

X Wasserstoff oder Fluor.

Die Herstellung der erfindungsgemäßen Verbindungen erfolgt nach folgenden Verfahren: Benzylalkohole

der allgemeinen Formel Ia

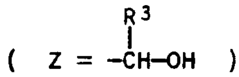

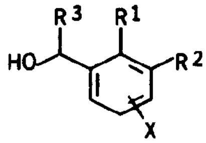

(Ia)

erhält man ausgehend von entsprechend substituierten Benzaldehyden der Formel Ib

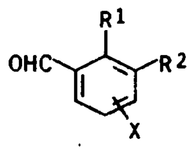

(Ib),

indem man sie umsetzt mit:

i) einem Reduktionsmittel im Fall von $R^3$=H,

ii) Blausäure oder einem Metallcyanid ggf. in Gegenwart einer Säure im Fall von $R^3$=CN

iii) Metallorganylen $MeR^3$ oder $R^3MeHal$ im Fall von $R^3$ = $C_2$-$C_4$-Alkinyl, $C_2$-$C_4$-Alkenyl oder $C_1$-$C_4$-Alkyl, wobei Me für ein Alkali-, Erdalkali- oder Übergangsmetall und Hal für Halogen steht.

Als Reduktionsmittel kommen alle gängigen Reduktionsmittel, die Benzaldehyde in Benzylalkohole überführen, in Frage (Houben-Weyl, Methoden der org. Chemie, Band VI/1b, S. 1 - 500, Ausgabe 1984, Georg Thieme Verlag, Stuttgart). Neben der katalytischen Hydrierung, nichtmetallischen Reduktionsmitteln und Metallhydriden sind besonders komplexe Metallhydride wie z.B. Lithiumaluminiumhydrid oder Natriumborhydrid geeignet. Kathodische- und photochemische Reduktion kommen ebenfalls in Frage.

Zur Herstellung der Cyanhydrine werden die Benzaldehyde mit Blausäure, in situ aus Metallcyaniden erzeugter Blausäure oder Metallcyaniden in Gegenwart von Alkalihydrogensulfitlösung umgesetzt, wobei gegebenenfalls basische Katalysatoren wie Kaliumcarbonat oder Phasentransferkatalysatoren wie z.B. Benzyltriethylammoniumchlorid zugegeben werden.

Als Metallcyanide werden bevorzugt Alkalimetallcyanide wie z.B. Natrium- oder Kaliumcyanid verwendet.

Die Umsetzung erfolgt in an sich bekannter Weise, z.B. wie in Houben-Weyl, Methoden der org. Chemie, Band VIII, S. 274-278, Ausgabe 1952 und Band E5, S. 1413ff, Ausgabe 1985 beschrieben.

Als Metallorganyle eignen sich die entsprechenden metallorganischen Verbindungen, insbesondere Lithiumorganylverbindungen $LiR^3$ wie Methyllithium, Ethyllithium, Butyllithium oder die entsprechenden Grignardverbindungen $R^3MgHal$, worin Hal für Chlor, Brom oder Iod steht, z.B. Methylmagnesiumbromid, Ethylmagnesiumchlorid, Propylmagnesiumiodid oder Vinylmagnesiumiodid.

Die Umsetzung mit Metallorganylen kann in an sich bekannter Weise, z.B. wie in Houben-Weyl, Methoden org. Chemie, Band 13/2a, S. 285ff, 1973 beschrieben in einem inerten organischen Lösungsmittel wie Ether oder Tetrahydrofuran unter Schutzgas durchgeführt werden, so daß sich nähere Ausführungen hierzu erübrigen.

Die Herstellung der Benzaldehyde Ib erfolgt in der Weise, daß man entsprechend substituierte Benzonitrile Ic

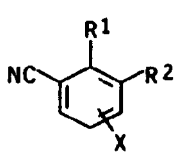

(Ic)

mit einem Reduktionsmittel zu Aldiminen II

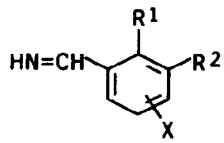

(II)

umsetzt und diese in an sich bekannter Weise verseift.

Diese Reaktionssequenz ist im folgenden Reaktionsschema dargestellt:

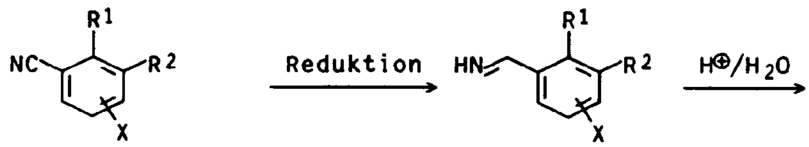


Ib

Ic II

Neben Wasserstoff (katalytische Hydrierung) und Tetrachlorozinn(II)-säure sind als Reduktionsmittel besonders Aluminiumhydride wie z.B. Diisopropylaluminiumhydrid geeignet (vgl. Houben-Weyl, Methoden der organischen Chemie, Band E3, S. 476-488, Georg Thieme Verlag, Stuttgart). Die Hydrolyse der Aldimine II erfolgt in der Regel durch Behandlung mit verdünnten oder konzentrierten Mineralsäuren wie Salzsäure. Bei empfindlichen aldehyden empfiehlt sich die Verwendung von gepufferter Essigsäure.

Die Isolierung der Aldimine II ist nicht unbedingt erforderlich. Die Aldimine können vorteilhaft ohne Aufarbeitung und Reinigung sofort zu den Benzaldehyden Ib hydrolysiert werden.

Zur Herstellung der Benzonitrile der allgemeinen Formel Ic

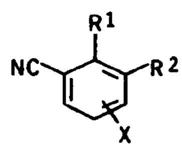

(Ic)

werden Verbindungen der allgemeinen Formel III

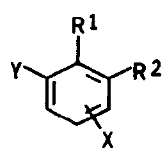

(III),

in der Y für Chlor oder Brom steht, mit Metallcyaniden in einem organischen Lösungsmittel gemäß folgender Reaktionsgleichung umgesetzt.

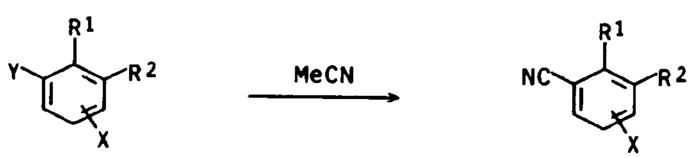


III Ic

Als Metallcyanide (MeCN) eignen sich Alkali-, Erdalkali- und Schwermetallcyanide (HOuben-Weyl, Methoden der organischen Chemie, Band E5, S. 1447-1467, Ausgabe 1985, Georg Thieme Verlag, Stuttgart). Besonders vorteilhaft ist die Verwendung von Kupfer(I)-cyanid. Die Umsetzung verläuft gut in aprotischen, polaren Lösungsmitteln. Besonders geeignet sind Dimethylformamid, Pyridin, 1-Methyl-2-pyrrolidon und Phosphorsäure-tris-(dimethylamid).

Die Reaktion wird vorteilhaft bei Temperaturen zwischen 100 und 250°C durchgeführt. Bei Verwendung von Kupfer(I)-cyanid erfolgt die Aufarbeitung des Reaktionsgemisches (Zerstörung der primär gebildeten Nitril/Kupferhalogenid/Kupfercyanid-Komplexe) entweder mit Eisen(III)-chlorid/Salzsäure, 1,2-Diamino-ethan oder Natriumcyanid. Vorteilhaft ist die Verwendung von 1,2-Diamino-ethan.

Einzelne Vertreter der Verbindungen III wie 2-Chlor-6-n-butyl-toluol, 3-Chlor-2-methylstyrol, 1-chlor-2-methyl-3-(1'-propenyl)benzol, 2,3-Dimethylchlorbenzol, 2,3-Dimethyl-brombenzol, 4-Fluor-2,3-dimethyl-brombenzol, 1,2-dichlor-3,4-dimethylbenzol, 1,5-dichlor-2,3-dimethylbenzol und 1,4-Dichlor-2,3-dimethylbenzol sind bekannt aus US-Patent 4 538 003; EP 80 359; J. Med. Chem. 28 (10), 1436-40; J. Chromatogr., 370 (3), 355-76 (1986); Gazz. Chim. Hal., 103 (8-9), 1019-25 (1973) oder Chem.-Ztg. 103 (1), 1-7 (1979).

Ein allgemeiner Syntheseweg zur Herstellung der Verbindungen der Formel III, in der $R^1$, $R^2$, X und Y die oben angegebene Bedeutung haben geht von Dichlor-, Dibrom-, Dibromchlor-, Dibromfluor- oder Dichlorfluorbenzolderivaten Va aus und wird durch folgendes Reaktionsschema verdeutlicht:

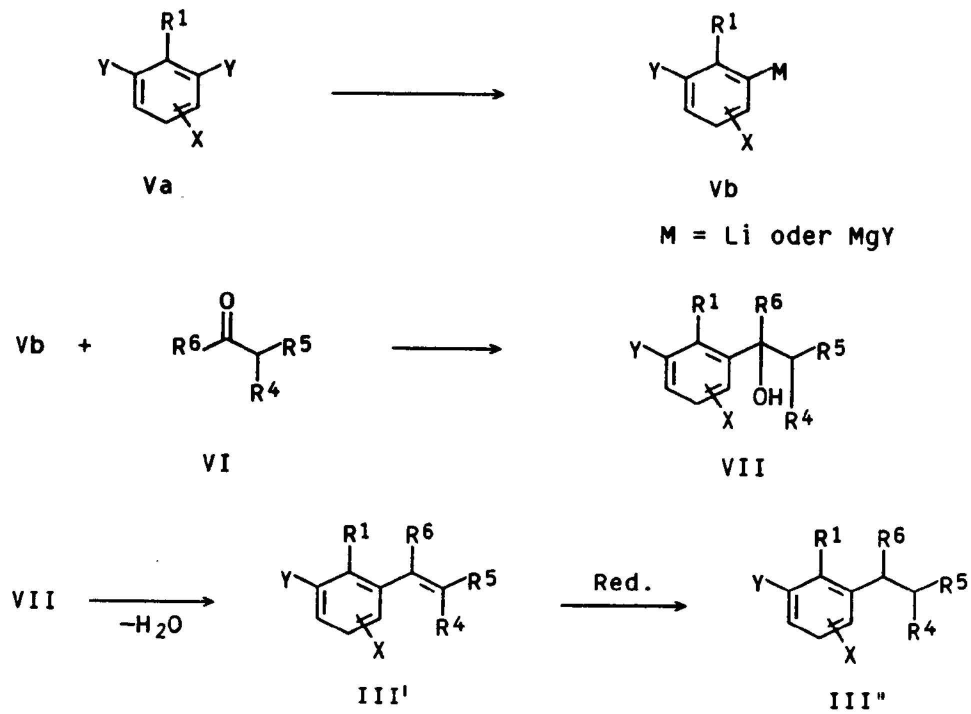


In diesem Reaktionsschema entsprechen die Seitenketten

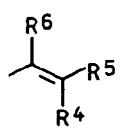 bzw. 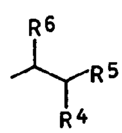 dem Rest $R^2$.

$R^4$, $R^5$ und $R^6$ haben die Bedeutung Wasserstoff, verzweigtes oder unverzweigtes Alkyl oder verzweigtes oder unverzweigtes Alkenyl. $R^4$ und $R^6$ bzw. $R^5$ und $R^6$ können auch zu einem Ring geschlossen sein, der gegebenenfalls mit $C_1$-$C_5$-Alkylresten substituiert ist oder $R^4$, $R^5$ und $R^6$ derart gestaltet, daß ein bicyclisches Keton vorliegt, welches gegebenenfalls mit $C_1$-$C_5$-Alkylresten substituiert ist.

Di- bzw. Trihalogenbenzole der allgemeinen Formel Va, in der $R^1$ Methyl oder Ethyl, X Wasserstoff, Chlor oder Fluor und Y Chlor oder Brom bedeuten, werden in die Monometallorganyle der allgemeinen Formel Vb überführt (lithiumorganische Verbindung oder Grignardverbindung), wobei X, Y und $R^1$ die für Va angegebene Bedeutung haben und M für Lithium oder MgY steht.

Zur Herstellung der Lithiumorgano- bzw. Grignardverbindungen kommen die gängigen Herstellverfahren, die von Arylhalogeniden ausgehen, in Frage (vgl. Houben-Weyl, Methoden der org. Chemie, Band XIII/1. S. 134 ff, Ausgabe 1970 und Band XIII/2a, S. 54 ff. Ausgabe 1973, Georg Thieme Verlag, Stuttgart). Die Synthese der Grignardverbindungen erfordert im Falle, daß Y für Chlor und X für Wasserstoff steht, höhere Reaktionstemperaturen. Besonders bewährt hat sich die Verwendung von Tetrahydrofuran bei Siedetemperatur.

Die metallorganischen Verbindungen Vb werden mit Carbonylverbindungen VI, in denen $R^4$ bis $R^6$ die oben

genannte Bedeutung haben, zu Benzylalkoholen VII umgesetzt. Diese werden zu Styrolen der allgemeinen Formel III′ dehydratisiert, wobei die in Houben-Weyl (Methoden der org. Chemie, Band V/Ib, S. 45 ff, Ausgabe 1972, Georg-Thieme-Verlag, Stuttgart) beschriebenen Methoden in Frage kommen. Vorteilhaft ist die Verwendung saurer Dehydratisierungsmittel, insbesondere von Oxalsäure oder p-Toluolsulfonsäure, bei gleichzeitiger Entfernung des gebildeten Wassers mittels eines Abscheiders.

Die auf die vorstehend beschriebene Art und Weise erhaltenen Styrolderivate III′ werden entweder direkt zur Herstellung der Benzonitrile der allgemeinen Formel Ic eingesetzt oder zuvor durch Reduktion in Verbindungen der allgemeinen Formel III″ umgewandelt. Dies kann sowohl durch nicht katalytische Reduktion (z.B. mit Ethanol und Natrium) als auch durch katalytische Hydrierung erfolgen (vgl. Houben-Weyl, Methoden der org. Chemie, Band V/1a, S. 405 ff, Ausgabe 1970, Georg Thieme Verlag, Stuttgart). Als Katalysatoren kommen z.B. $PtO_2$, Raney-Nickel, Pd/Kohle, $Pd/CaCO_3$, Kupferchromit oder $Pd/Al_2O_3$ in Frage. Besonders bewährt hat sich die Verwendung von Pd/Kohle. Als Lösungsmittel eignen sich: Alkohole wie z.B. Methanol, Ethanol oder Isopropanol; Ether wie z.B. Tetrahydrofuran oder Dioxan; Ester wie z.B. Essigsäureethylester. Vorteilhaft ist die Verwendung von Ethanol. Die katalytische Hydrierung wird in der Regel bei Raumtemperatur und Drucken zwischen 1 und 150 bar durchgeführt. Als Nebenprodukte entstehen oft geringe Mengen von Verbindungen bei denen zusätzlich ein Halogen/Wasserstoff-Austausch stattgefunden hat.

Verbindungen der allgemeinen Formel III

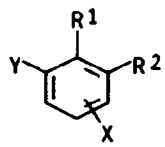

(III),

in der $R^2$ einen tertiären Alkylrest bzw. tertiären Alkenylrest bedeutet, können ausgehend von Verbindungen der allgemeinen Formel Va hergestellt werden.

Folgendes Reaktionsschema soll anhand eines Beispiels den Syntheseweg verdeutlichen:

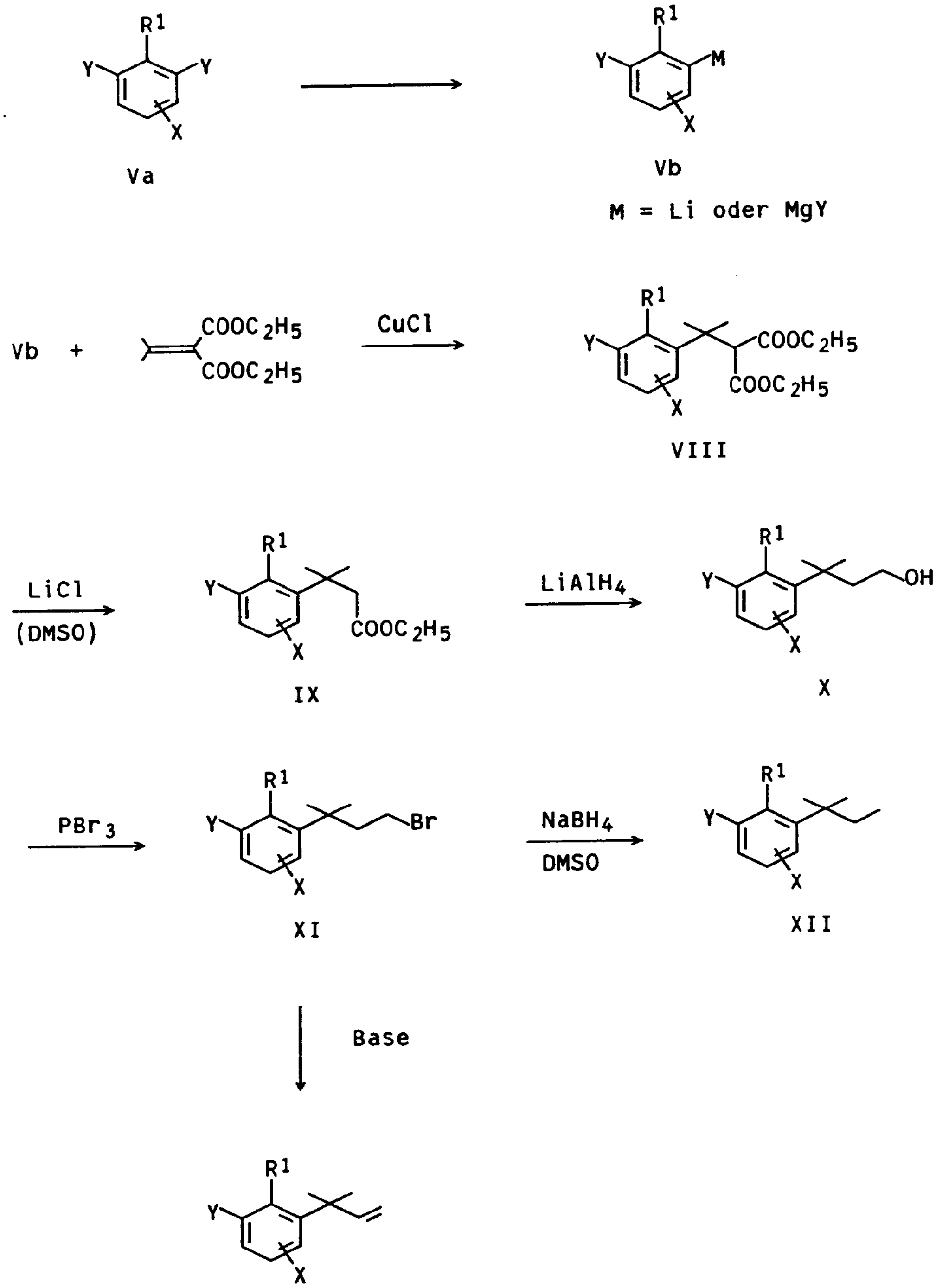


Va

Vb

M = Li oder MgY

VIII

IX

X

XI

XII

XIII

Verbindungen der allgemeinen Formel I

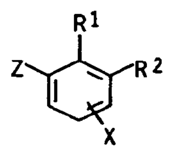

(I),

in der $R^2$ die Bedeutung ggf. substituiertes Cyclopropyl hat, können auf verschiedenen Wegen erhalten werden:

a) nach den in den Ansprüchen 3 - 5 beschriebenen Verfahren, wobei die erforderlichen Ausgangspunkte

XV durch Addition von Dihalogencarbenen an Verbindungen der allgemeinen Formel III' und anschließende Dehalogenierung (z.B. mit Tri-n-butylzinnhydrid) gewonnen werden können.

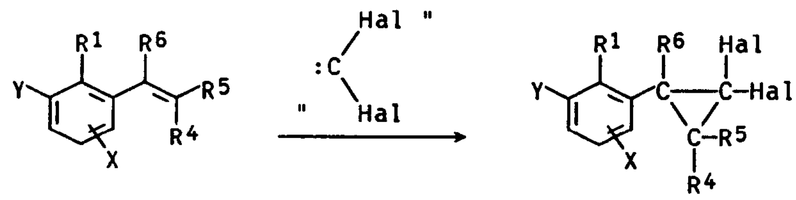

III'

XIV

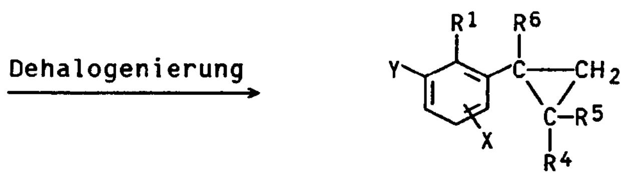


XV

($R^4$, $R^5$, $R^6$ = H oder $C_1$-$C_5$-Alkyl)

b) Dihalogencarbenaddition und anschließende Dehalogenierung werden auf einer späteren Stufe (entsprechend substituierte Benzonitrile, Benzaldehyde oder Benzylalkohole) durchgeführt, wobei Z, X, $R^1$ und $R^4$ - $R^6$ die oben angegebene Bedeutung haben:

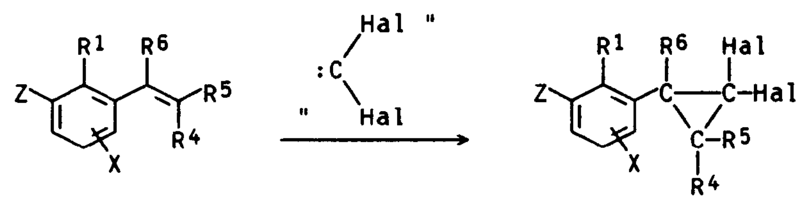

XVI

XVII

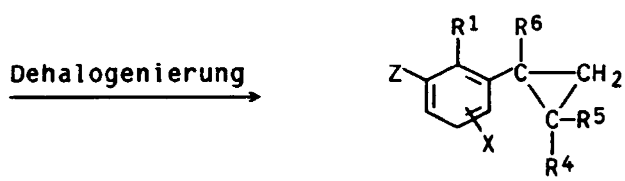


XVIII

Im Falle von Z = -CHO und

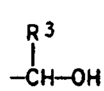

ist es vorteilhaft, zuvor Schutzgruppen für diese funktionellen Gruppen einzuführen (z.B. Acetal, Tetrahydropyranylether) und diese nach der Dehalogenierung in an sich bekannter Weise wieder abzuspalten.

Die Herstellung der erfindungsgemäßen Verbindungen bzw. deren Vorprodukte erfolgt gemäß den Beschreibungen der folgenden Beispiele bzw. durch entsprechende Abwandlung dieser Beispiele.

Beispiel 1

Herstellung von Benzylalkoholen VII

1-(3′-Chlor-2′-methylphenyl)cyclohexanol

Unter Stickstoffatmosphäre werden 96 g (4 mol) Mg (Späne) in 60 ml absolutem THF (Tetrahydrofuran) vorgelegt. Bei 65 °C fügt man 1 ml 1,2-Dibromethan hinzu und tropft dann eine Lösung von 644 g (4 mol) 2,6-Dichlortoluol in 1,5 l absolutem THF innerhalb von 2 1/4 Stunden hinzu. Anschließend rührt man 4 Stunden unter Rückfluß, kühlt auf Raumtemperatur und fügt unter $N_2$ 352,8 g (3,6 mol) Cyclohexanon in 250 ml absolutem THF hinzu. Nach erfolgter Umsetzung wird das Grignard-Reaktionsgemisch wie üblich wäßrig aufgearbeitet und das Lösungsmittel durch Destillation im Vakuum weitgehend entfernt. Der Rückstand wird andestilliert (30 bis 107°C/0,27 mbar). Das verbleibende Rohprodukt (676 g), welches bereits 90 % 1-(3′-Chlor-2′-methylphenyl)cyclohexanol enthält, kann mittels Säulenchromatographie (Fließmittel: Toluol) weiter gereinigt werden.

300-MHz-NMR-Spektrum in $CDCl_3$

δ [ppm] = 1,58 - 2,0(10H); 2,65(3H); 7,04(1H); 7,23 - 7,34(2H)

Nach dem vorstehend beschriebenen Verfahren wurden z.B. folgende Alkohole hergestellt, die durch die Angabe physikalischer Daten näher charakterisiert sind:

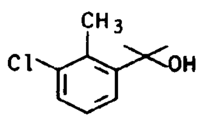

Smp.: 55-59°C

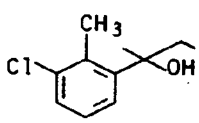

300-MHz-NMR-Spektrum in $ClCl_3$:

δ [ppm] = 0,82(3H); 1,62(3H); 1,86-2,07[2H+1H(OH)]; 2,59(3H); 7,06(1H); 7,29(1H); 7,36(1H)

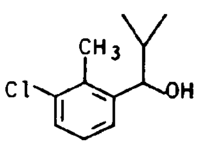

300-MHz-NMR-Spektrum in $ClCl_3$:

δ [ppm] = 0,84(3H); 0.96(3H); 1,83-1,96(1H); 2,19(1H); 2,32 (3H); 4,63(1H); 7,1(1H); 7,23-7,33(2H)

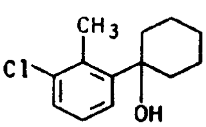

Smp.: 113°C

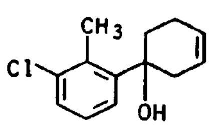

Smp.: 90 bis 93°C

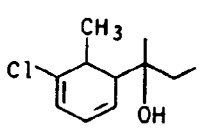

Smp.: 52 bis 55°C

Beispiele 2 bis 31

Herstellung von Halogenbenzolderivaten III

2-Methyl-3-Isopropenyl-chlorbenzol

190 g 3-Chlor-2-methyl-alpha,alpha-dimethylbenzylalkohol werden mit 1,5 l Toluol und 370 g Oxalsäure am Wasserabscheider unter Rückfluß erhitzt, bis sich kein Wasser mehr abscheidet. Die Oxalsäure wird abgesaugt und das Filtrat mit Natriumhydrogencarbonat-Lösung gewaschen, über Natriumsulfat getrocknet und eingeengt. Nach fraktionierter Destillation des Rückstands erhält man 91,5 g der gewünschten Verbindung die in Tabelle 1 als Beispiel 2 aufgeführt ist.

300-MHz-NMR-Spektrum in $CDCl_3$:

δ [ppm] = 1,98(3H); 2,32(3H); 4,82(1H); 5,17(1H); 6,95-7,07(2H); 7,23(1H);

3-Cyclohexyl-2-methyl-chlorbenzol

103 g 1-(3′-Chlor-2′-methylphenyl)cyclohexen werden in 1 200 ml Ethanol gelöst und mit 5 g Pd/Kohle versetzt. Bei Raumtemperatur und 80 bar Wasserstoffdruck wird 8 Stunden lang hydriert. Man filtriert den Katalysator vom Reaktionsaustrag ab und engt die Lösung ein. Nach fraktionierter Destillation erhält man 51,8 g 3-Cyclohexyl-2-methyl-chlorbenzol (Beispiel 3 in Tabelle 1).

300-MHz-NMR-Spektrum in $CDCl_3$:

δ [ppm] = 1,2-1,49(5H); 1,7-1,92(5H); 2,36(3H); 2,72(1H); 7,01-7,2 (3H).

Analog den vorstehend beschriebenen Beispielen 2 bzw. 3 wurden die in der folgenden Tabelle 1 genannten Verbindungen hergestellt, die durch die Angabe physikalischer Daten näher charakterisiert sind; die übrigen Verbindungen der Tabelle 1 können unter Verwendung entsprechender Ausgangsstoffe leicht erhalten werden.

Tabelle 1

Halogenbenzolderivate der Formel III

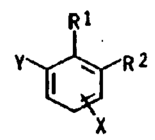

III

| Beispiel | Y | $R^1$ | $R^2$ | X | Physikalische Daten |
|---|---|---|---|---|---|
| 2 | Cl | $CH_3$ | Isopropenyl | H | Kp. 54-55°C/0,13 mbar |
| 3 | Cl | $CH_3$ | Cyclohexyl | H | Kp. 88-93°C/0,013 bar |
| 4 | Cl | $CH_3$ | n-Propyl | H | |
| 5 | Cl | $CH_3$ | $CH_3$<br>$-C=CH-CH_3$ | H | |
| 6 | Br | $CH_3$ | Cyclopentyl | H | |
| 7 | Cl | $CH_3$ | 1-Cyclohexenyl | H | Kp. 95-98°C/0,2 mbar<br>$n_D^{23}$= 1,5501 |
| 8 | Br | $C_2H_5$ | Isopropyl | H | |
| 9 | Cl | $CH_3$ | Cyclohexyl | 5-F | |
| 10 | Cl | $CH_3$ | Isopropyl | 5-Cl | |
| 11 | Cl | $CH_3$ | Isopropyl | H | Kp. 55°C/0,27 mbar |
| 12 | Br | $CH_3$ | Isopropyl | H | |
| 13 | Cl | $C_2H_5$ | Isopropenyl | H | |
| 14 | Br | $CH_3$ | Isopropenyl | H | |
| 15 | Cl | $CH_3$ | sec-Butyl | H | Kp. 58-60°C/0,13 mbar |
| 16 | Cl | $CH_3$ | $CH_3$<br>$-C-C(CH_3)_3$ | H | |

Tabelle 1 (Fortsetzung)

| Beispiel | Y | $R^1$ | $R^2$ | X | Physikalische Daten |
|---|---|---|---|---|---|
| 17 | Cl | $CH_3$ | 1-Cyclopentenyl | H | |
| 18 | Cl | $CH_3$ | 2,5-Norbornadien-2-yl | H | |
| 19 | Cl | $CH_3$ | 2-Norbornen-2-yl | H | Kp. 113°C/0,06 mbar<br>$n_D^{21}$= 1,5702 |
| 20 | Cl | $CH_3$ | 2-Norbornen-2-yl | 5-F | |
| 21 | Br | $CH_3$ | 2-Norbornen-2-yl | H | |
| 22 | Cl | $C_2H_5$ | Norborn-2-yl | H | |
| 23 | Cl | $CH_3$ | Norborn-2-yl | H | Kp 109-110°C/0,013 mbar |
| 24 | Cl | $CH_3$ | 3,5-Diethylcyclohexyl | H | |
| 25 | Cl | $CH_3$ | 1,3-Cyclohexadienyl | H | |
| 26 | Br | $CH_3$ | $C_2H_5$<br>\|<br>$-C=CH-CH_3$ | | |
| 27 | Cl | $CH_3$ | $C_2H_5$<br>\|<br>$-C=CH-CH_3$ | H | Kp. 70-72°C/0,013 mbar |
| 28 | Cl | $C_2H_5$ | $C_2H_5$<br>\|<br>$-C=CH-CH_3$ | H | |
| 29 | Cl | $CH_3$ | $CH_2-CH_2-CH=CH_2$<br>\|<br>$-C=CH_2$ | H | |
| 30 | Cl | $CH_3$ | $CH_2-CH(CH_3)_2$<br>\|<br>$-C=CH_2$ | H | |
| 31 | Cl | $CH_3$ | $-CH(C_2H_5)_2$ | H | Kp. 60-62°C/0,013 mbar |

Beispiele 32 bis 65 und 131

Herstellung von Benzonitrilen Ic

3-(1′-Cyclohexenyl)-2-methylbenzonitril

250 g (0,6 mol) 1-(3′-Chlor-2′-methylphenyl)cyclohexen, 600 ml 1-Methyl-2-pyrrolidon und 63 g wasserfreies Kupfer(I)-cyanid werden unter Rühren 35 Stunden zum Sieden erhitzt. Das Reaktionsgemisch wird in eine Lösung von 500 ml Ethylendiamin in 1,5 l Wasser gegossen, 45 Minuten bei 50°C gerührt und mehrmals mit Toluol extrahiert. Die vereinigten organischen Phasen schüttelt man mit 10 %iger Natriumcyanid-Lösung aus, trocknet über $Na_2SO_4$ und engt ein. Nach säulenchromatographischer Reinigung über Kieselgel mit Toluol/Cyclohexan (3/7) als Elutionsmittel erhält man 117,8 g Nitril (Schmp. 54 - 57°C).

250-MHz-NMR-Spektrum in $CDCl_3$:

δ [ppm] = 1,72(4H); 2,13(4H); 2,46(3H); 5,55(1H); 7,17-7,31(2H); 7,49(1H);

Nach dem voranstehend beschriebenen Verfahren wurden die in der Tabelle 2 genannten erfindungsgemäßen Benzonitrile hergestellt, die durch die Angabe physikalischer Eigenschaften näher charakterisiert sind; die übrigen Verbindungen der Tabelle 2 können unter Verwendung entsprechender Ausgangsstoffe leicht erhalten werden.

Tabelle 2
Benzonitrile der Formel Ic

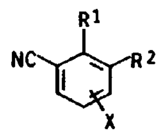

Ic

| Beispiel | $R^1$ | $R^2$ | X | Physikalische Eigenschaften |
|---|---|---|---|---|
| 32 | $CH_3$ | 1-Cyclohexenyl | H | Schmp.: 54 bis 57°C |
| 33 | $CH_3$ | n-Propyl | H | |
| 34 | $CH_3$ | n-Butyl | H | |
| 35 | $CH_3$ | $-CH(C_2H_5)_2$ | H | $n_D^{23}$: 1,5119 |
| 36 | $C_2H_5$ | Isopropyl | H | |
| 37 | $CH_3$ | Isopropyl | H | 300-MHz-NMR-Spektrum in $CDCl_3$:<br>δ [ppm] = 1,23(6H); 2,55(3H); 3,19(1H); 7,25(1H); 7,46(2H) |
| 38 | $CH_3$ | Isopropyl | 6-F | |
| 39 | $CH_3$ | $-C(C_2H_5)=CH_2$ | H | |
| 40 | $C_2H_5$ | Isopropenyl | H | |
| 41 | $CH_3$ | $-C(CH_3)=CH-CH_3$ | H | |
| 42 | $CH_3$ | Isopropenyl | H | 200-MHz-NMR-Spektrum in $CDCl_3$:<br>δ [ppm] = 2,02(3H); 2,52(3H); 4,89(1H); 5,3(1H); 7,22-7,4(2H); 7,56(1H) |

Tabelle 2 (Fortsetzung)

| Beispiel | $R^1$ | $R^2$ | X | Physikalische Eigenschaften |
|---|---|---|---|---|
| 43 | $CH_3$ | Isopropenyl | 5-Cl | |
| 44 | $CH_3$ | $CH_3$<br>$-C=C(CH_3)_2$ | H | |
| 45 | $CH_3$ | $i-C_3H_7$<br>$-C=CH_2$ | H | |
| 46 | $CH_3$ | $CH_2-CH_2-CH=CH_2$<br>$-C=CH_2$ | H | |
| 47 | $CH_3$ | sec. Butyl | H | 200-MHz-NMR-Spektrum in $CDCl_3$:<br>δ [ppm] = 0,84(3H); 1,2(3H); 1,61(2H); 2,54(3H); 2,94(1H); 7,2-7,31(1H); 7,43(2H) |
| 48 | $C_2H_5$ | sec. Butyl | H | |
| 49 | $CH_3$ | $i-C_3H_7$<br>$-CH-CH_3$ | H | |
| 50 | $C_2H_5$ | $CH(C_2H_5)_2$ | H | |
| 51 | $CH_3$ | Cyclopentyl | H | |
| 52 | $CH_3$ | Cyclohexyl | H | 300-MHz-NMR-Spektrum in $CDCl_3$:<br>δ [ppm] = 1,2-1,5(5H); 1,71-1,93(5H); 2,55(3H); 2,75(1H); 7,23 (1H); 7,42 (2H) |
| 53 | $CH_3$ | Cyclopropyl | H | |
| 54 | $CH_3$ | 1-Cyclopentenyl | H | |
| 55 | $CH_3$ | 1,3-Cyclohexadienyl | H | |
| 56 | $CH_3$ | 2-Norbornen-2-yl | H | |

Tabelle 2 (Fortsetzung)

| Beispiel | $R^1$ | $R^2$ | X | Physikalische Eigenschaften |
|---|---|---|---|---|
| 57 | $CH_3$ | Norborn-2-yl | H | Schmp. 74 bis 76°C |
| 58 | $C_2H_5$ | Cyclohexyl | H | |
| 59 | $CH_3$ | 2,5-Norbornadien-2-yl | H | |
| 60 | $CH_3$ | Cycloheptyl | H | |
| 61 | $CH_3$ | $CH_3$<br>\|<br>$-CH-CH_2-CH_2-CH_3$ | H | |
| 62 | $CH_3$ | $C_2H_5$<br>\|<br>$-C{=}CH-CH_3$ | H | $n_D^{22}$: 1,5254 |
| 63 | $CH_3$ | $C_2H_5$<br>\|<br>$-C{=}CH-CH_3$ | 5-F | |
| 64 | $C_2H_5$ | $C_2H_5$<br>\|<br>$-C{=}CH-CH_3$ | H | |
| 65 | $CH_3$ | $-CH(i-C_3H_7)_2$ | H | |
| 131 | $CH_3$ | $C_2H_5$<br>\|<br>$-C(CH_3)_2$ | H | 300-MHz-NMR-Spektrum in $CDCl_3$:<br>δ [ppm] = 0,65(3H); 1,41(6H); 1,83(2H); 2,72(3H); 7,2(1H); 7,5(2H) |

Beispiele 66 bis 94 und 132

Herstellung der Benzaldehyde Ib

3-(1′-Cyclohexenyl)-2-methylbenzaldehyd

Zu einer Lösung von 113 g (0,574 mol) 3-(1′-Cyclohexenyl)-2-methylbenzonitril in 1 000 ml trockenem Toluol werden unter Stickstoff bei 20-30°C vorsichtig 460 ml (0,69 mol) Diisobutylaluminiumhydrid-Lösung (25 %ige Lösung in Toluol) getropft. Man rührt 4 Stunden bei Raumtemperatur nach und zersetzt überschüssiges Diisobutylaluminiumhydrid mit 120 ml Methanol. Nach Zugabe von 1 000 ml 10 %iger Salzsäure wird über Nacht bei Raumtemperatur gerührt. Die organische Phase wird abgetrennt und die wäßrige Phase mehrmals mit Toluol extrahiert. Die vereinigten organischen Phasen werden nach Waschen mit Wasser über $Na_2SO_4$ getrocknet. Nach dem Entfernen des Lösungsmittels ergeben sich 109 g Aldehyd ($n_D^{22}$:1,5621).

300-MHz-NMR-Spektrum in $CDCl_3$:

$\delta$ [ppm] = 1,63-1,82(4H); 2,14(4H); 2,59(3H); 2,55(1H); 7,28(2H); 7,67(1H), 10,29(1H)

Nach dem voranstehend beschriebenen Verfahren wurden die in der folgenden Tabelle 3 genannten erfindungsgemäßen Benzaldehyde hergestellt, die durch die Angabe physikalischer Eigenschaften näher charakterisiert sind; die übrigen Verbindungen der Tabelle 3 können unter Verwendung entsprechender Ausgangsstoffe leicht erhalten werden.

Tabelle 3
Benzaldehyde

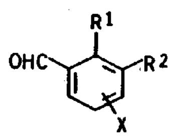

Ib

| Beispiel | $R^1$ | $R^2$ | X | Physikalische Eigenschaften |
|---|---|---|---|---|
| 66 | $CH_3$ | 1-Cyclohexenyl | H | $n_D^{22}$= 1,5621 |
| 67 | $CH_3$ | Cyclopentyl | H | |
| 68 | $CH_3$ | 1-Cyclopentenyl | H | |
| 69 | $C_2H_5$ | Cycloheptyl | H | |
| 70 | $CH_3$ | Cyclopropyl | H | |
| 71 | $CH_3$ | Cyclohexyl | 5-Cl | |
| 72 | $CH_3$ | Cyclohexyl | H | Schmp. 42-45°C |
| 73 | CH§ | Norbornen-2-yl | H | |
| 74 | CH§ | 2,5-Norbornadien-2-yl | H | |
| 75 | $CH_3$ | $-\overset{C_2H_5}{\overset{\vert}{C}}=CH_2$ | H | |

Tabelle 3 (Fortsetzung)

| Beispiel | $R^1$ | $R^2$ | X | Physikalische Eigenschaften |
|---|---|---|---|---|
| 76 | $CH_3$ | 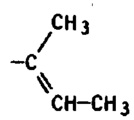 | H | |
| 77 | $CH_3$ | $-CH(C_2H_5)_2$ | H | 300-MHz-NMR-Spektrum in $CDCl_3$: δ [ppm] = 0,77(6H); 1,56(2H); 1,72(2H); 2,64(3H); 2,91(1H); 7,37(2H); 7,63(1H); 10,33(1H) |
| 78 | $CH_3$ | 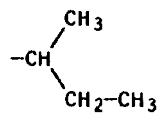 | H | 250-MHz-NMR-Spektrum in $CDCl_3$: δ [ppm] = 0,86(3H); 1,22(3H); 1,62(2H); 2,64(3H); 3,04(1H); 7,33(1H); 7,44(1H); 7,63(1H); 10,35(1H) |
| 79 | $C_2H_5$ | 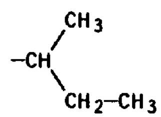 | H | |
| 80 cis/trans | $CH_3$ | 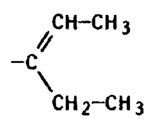 | H | 200-MHz-NMR-Spektrum in $CDCl_3$: δ[ppm] = 0,89 und 0,97 (3H); 1,36 und 1,8 (3H); 2,25 und 2,37 (2H); 2,52 und 2,57 (3H); 5,33 und 5,64 (1H); 7,22-7,4 (2H); 7,73 (1H) |
| 81 | $CH_3$ | 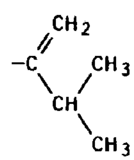 | H | |

Tabelle 3 (Fortsetzung)

| Beispiel | $R^1$ | $R^2$ | X | Physikalische Eigenschaften |
|---|---|---|---|---|
| 82 | $CH_3$ | 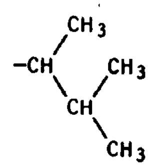 | H | |
| 83 | $CH_3$ | 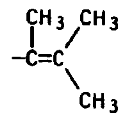 | H | |
| 84 | $CH_3$ | Isopropyl | H | 300-MHz-NMR-Spektrum in $CDCl_3$:<br>$\delta$ [ppm] = 1,27(6H); 2,66(3H); 3,3(1H); 7,33(1H); 7,5(1H); 7,65(1H); 10,32(1H) |
| 85 | $CH_3$ | Isopropyl | 5-F | |
| 86 | $CH_3$ | n-Propyl | H | |
| 87 | $CH_3$ | n-Butyl | H | |
| 88 | $CH_3$ | 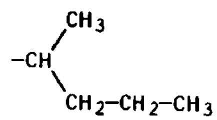 | H | |
| 89 | $C_2H_5$ | Isopropenyl | H | |
| 90 | $CH_3$ | Isopropenyl | H | 200-MHz-NMR-Spektrum in $CDCl_3$:<br>$\delta$ [ppm] = 2,01(3H); 2,6(3H); 4,85(1H); 5,26(1H); 7,35(2H); 7,73(1H); 10,33(1H) |
| 91 | $CH_3$ | 1,3-Cyclohexadienyl | H | |
| 92 | $CH_3$ | Vinyl | H | |

Tabelle 3 (Fortsetzung)

| Bei-spiel | $R^1$ | $R^2$ | X | Physikalische Eigenschaften |
|---|---|---|---|---|
| 93 | $CH_3$ | 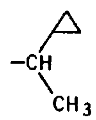 | H | |
| 94 | $CH_3$ | Norborn-2-yl | H | 200 MHz-NMR-Spektrum in $CDCl_3$: δ[ppm] = 1,17-174 (7H); 1,96 (1H); 2,39 (2H); 2,68 (3H); 3,53 (1H); 7,37 (1H); 7,52 (1H); 7,69 (1H); 10,38 (1H) |
| 132 | $CH_3$ | 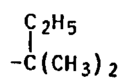 | H | 300-MHz-NMR-Spektrum in $CDCl_3$: δ [ppm] = 0,68(3H); 1,42(6H); 1,86(2H); 2,8(3H); 7,27(1H); 7,57(1H) 7,66(1H); 10,38(1H) |

Beispiele 95 bis 130

Herstellung der Benzylalkohole Ia

3-(1′-Cyclohexenyl)-2-methylbenzylalkohol

Zu einer Suspension von 2,3 g (0,06 mol) Natriumborhydrid in 120 ml Ethanol wird bei Raumtemperatur eine Lösung von 24 g (0,12 mol) 3-(1′-Cyclohexenyl)-2-methylbenzaldehyd in 120 ml Ethanol zugetropft. Nach 15 stündigem Rühren bei Raumtemperatur versetzt man die Mischung vorsichtig mit 320 ml 5 %iger Salzsäure und extrahiert mehrmals mit Ether. Die vereinigten Etherphasen werden mit 5 %iger Salzsäure und anschließend mit Wasser gewaschen, getrocknet und eingeengt. Man erhält 23 g 3-(1′-Cyclohexenyl)-2-methylbenzylalkohol (Fp. 62-64°C; Beispiel 95).

3-sec.-Butyl-2-methyl-(alpha-ethinyl)benzylalkohol

50 ml THF (absolut) werden unter Stickstoffatmosphäre bei 0°C mit Acetylen gesättigt. Unter weiterem Einleiten von Acetylen tropft man innerhalb von 45 Minuten 87 ml Methylmagnesiumchlorid-Lösung (1,5 molar) zu und rührt 30 Minuten bei 0°C. Bei -20°C wird eine Lösung von 15,3 g 3-sec.-Butyl-2-methylbenzaldehyd in 20 ml THF (abs.) eingetropft. Man rührt 2 Stunden bei -20°C, läßt über Nacht bei RT stehen und gießt die Reaktionsmischung in 300 ml Eiswasser. Nach Ansäuern mit verd. Salzsäure wird 3 mal mit Ether ausgeschüttelt. Die vereinigten Etherextrakte werden mit Wasser gewaschen, über Natriumsulfat getrocknet und eingeengt. Man erhält 16,6 g eines Öls (70 % gewünschte Verbindung), welches nach säulenchromatographischer Reinigung über Kieselgel mit Toluol als Fließmittel 5,1 g des reinen Benzylalkohols ergibt (Beispiel 96).

300-MHz-NMR-Spektrum in $CDCl_3$:

δ [ppm] = 0,84, (3H); 1,17(3H); 1,57(2H); 2,36(3H); 2,57(1H); 2,62(1H); 2,97(1H); 5,62(1H); 7,18(2H); 7,52(1H)

3-Cyclopropyl-2-methylbenzylalkohol

a) Dihalogencarbenaddition: 9,9 g (0,042 mol) (2-Methyl-3-vinylbenzyl)-(tetrahydro-2-pyranyl)ether werden mit 10 ml Methylenchlorid, 0,42 ml Ethanol, 0,14 g Benzyl-triethylammoniumchlorid und 20,9 g (0,083 mol) Bromoform versetzt. Nach Zugabe von 13,28 g (0,166 mol) eiskalter 50 %iger NaOH wird 1 Stunde bei Raumtemperatur und 8 Stunden bei 50°C intensiv gerührt. Die Reaktionsmischung gießt man in 300 ml Wasser und extrahiert 3 mal mit Methylenchlorid. Die vereinigten Extrakte werden getrocknet und eingeengt. Nach säulenchromatographischer Reinigung über Kieselgel mit Toluol als Fließmittel erhält man 9,1 g [3-(2′,2′-Dibromcyclopropyl)-2-methylbenzyl]-tetrahydro-2-pyranyl-ether.

250-MHz-NMR-Spektrum in $CDCl_3$:

δ [ppm] = 1,45-1,95(6H); 2,02(1H); 2,15(1H); 2,46(3H); 2,84(1H); 3,55(1H); 3,9(1H); 4,55(1H); 4,72(1H); 4,88(1H); 6,93(1H); 7,14(1H); 7,37(1H)

b) Dehalogenierung: 29,7 g (0,074 mol) der unter a) hergestellten Dibromcyclopropylverbindung werden mit 150 ml n-Hexan bei 0°C mit 45,2 g (0,155 mol) Tri-n-butylzinnhydrid versetzt. Es wird 1 Stunde bei Raumtemperatur und 10 Stunden unter Rückfluß gerührt. Man erhitzt nochmals 20 Stunden unter Rückfluß, wobei portionsweise noch insgesamt 15 g Tri-n-butylzinnhydrid zugegeben werden. Die abgekühlte Reaktionslösung wird eingeengt. Nach säulenchromatographischer Reinigung über Kieselgel mit Toluol als Fließmittel ergeben sich 17,1 g (3-Cyclopropyl-2-methylbenzyl)-(tetrahydro-2-pyranyl)ether.

200-MHz-NMR-Spektrum in $CDCl_3$:

δ [ppm] = 0,62(2H); 0,91(2H); 1,5-2,0(6H+1H); 1,94(3H); 3,6(1H); 3,97(1H); 4,52(1H); 4,76(1H); 4,87(1H); 7,02-7,21(2H); 7,29(1H)

c) Abspaltung der Schutzgruppe: 16,7 g (3-Cyclopropyl-2-methylbenzyl)(tetrahydro-2-pyranyl)ether in 180 ml Methanol werden mit 10,8 ml konzentrierter Salzsäure über Nacht bei Raumtemperatur gerührt. Unter Eiskühlung wird mit Natriummethylat-Lösung neutralisiert und anschließend eingeengt. Den Rückstand versetzt man mit Wasser und extrahiert mehrmals mit Ether. Die vereinigten Etherextrakte werden mit Wasser gewaschen, getrocknet und eingeengt. Das Rohrprodukt (11,5 g) wird über Kieselgel mit Toluol als Fließmittel säulenchromatographisch gereinigt. Man erhält 8,9 g 3-Cyclopropyl-2-methylbenzylalkohol (Beispiel 100)

300-MHz-NMR-Spektrum in $CDCl_3$:

δ [ppm] = 0,61(2H); 0,93(2H); 1,88(1H); 2,15(1H); 2,4(3H); 4,63(2H); 6,97-7,19(3H)

Nach den in den Herstellbeispielen 95, 96 und 100 beschriebenen Verfahren wurden die in der folgenden

Tabelle 4 genannten erfindungsgemäßen Benzylalkohole hergestellt, die durch die Angabe physikalischer Eigenschaften näher charakterisiert sind; die übrigen Verbindungen der Tabelle 4 können unter Verwendung entsprechender Ausgangsstoffe leicht erhalten werden.

Tabelle 4

Benzylalkohole der Formel Ia

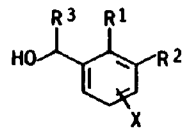

Ia

| Beispiel | $R^1$ | $R^2$ | $R^3$ | X | Physikalische Eigenschaften |
|---|---|---|---|---|---|
| 95 | $CH_3$ | 1-Cyclohexenyl | H | H | Fp. 62 bis 64°C |
| 96 | $CH_3$ | sec-Butyl | Ethinyl | H | 300-MHz-NMR Spectrum in $CDCl_3$: δ [ppm] = 0,84(3H); 1,17(3H); 1,57(2H); 2,36(3H); 2,57(1H); 2,62(1H); 2,97(1H); 5,62(1H); 7,18(2H); 7,52(1H) |
| 97 | $CH_3$ | Cyclopentyl | H | H | |
| 98 | $CH_3$ | 1-Cyclopentenyl | H | H | |
| 99 | $CH_3$ | 1-Cyclopentenyl | Ethinyl | H | |
| 100 | $CH_3$ | Cyclopropyl | H | H | 300-MHz-NMR-Spektrum in $CDCl_3$: δ [ppm] = 0,61(2H); 0,93(2H); 1,88(1H); 2,15(1H); 2,4(3H); 4,63(2H); 6,97-7,19(3H) |
| 101 | $CH_3$ | Cyclohexyl | H | 5-Cl | |
| 102 | $CH_3$ | Cyclohexyl | H | H | Schmp. 87-88°C |
| 103 | $CH_3$ | Cyclohexyl | Ethyl | H | |
| 104 | $CH_3$ | 2-Norbornen-2-yl | H | H | |
| 105 | $CH_3$ | 2,5-Norbornadien-2-yl | H | H | |
| 106 | $CH_3$ | Norborn-2-yl | H | H | 200 MHz-NMR-Spektrum in $CDCl_3$: δ[ppm] = 1,21-1,73 [7H+1H(OH)]; 1,94 (1H); 2,37 (3H+2H); 3,5 (1H); 4,77 (2H); 7,26 (3H) |

Tabelle 4 (Fortsetzung)

| Bei-spiel | $R^1$ | $R^2$ | $R^3$ | X | Physikalische Eigenschaften |
|---|---|---|---|---|---|
| 107 | $CH_3$ | $-CH(C_2H_5)_2$ | H | H | 200-MHz-NMR-Spektrum in $CDCl_3$:<br>δ [ppm] = 0,79(6H); 1,49-1,8(4H); 1,7(1H);<br>2,34(3H); 2,85(1H); 4,74(2H); 7,21(3H); |
| 108 | $CH_3$ | Isopropyl | H | H | 300-MHz-NMR-Spektrum in $CDCl_3$:<br>δ [ppm] = 1,22(6H); 2,24(1H); 2,31(3H); 3,22(1H);<br>4,63(2H); 7,12-7,24(3H) |
| 109 | $CH_3$ | Isopropyl | $CH_3$ | H | 300-MHz-NMR-Spektrum in $CDCl_3$:<br>δ [ppm] = 1,22(6H); 1,43(3H); 2,27(3H); 2,45(1H);<br>3,21(1H); 5,14(1H); 7,19(2H); 7,35(1H); |
| 110 | $CH_3$ | Isopropyl | H | 5-Cl | |
| 111 | $CH_3$ | Isopropyl | Ethinyl | H | |
| 112 | $CH_3$ | $CH_3$<br>$-CH-CH(CH_3)_2$ | H | H | |
| 113 | $CH_3$ | n-Propyl | H | H | |
| 114 | $CH_3$ | n-Butyl | H | H | |
| 115 | $CH_3$ | Isopropenyl | H | H | 200-MHz-NMR-Spektrum in $CDCl_3$:<br>δ [ppm] = 1,83(1H); 2,0(3H); 2,28(3H); 4,71(2H);<br>4,82(1H); 5,2(1H); 7,05-7,33(3H) |
| 116 | $CH_3$ | Isopropenyl | Ethinyl | H | |
| 117 | $CH_3$ | Isopropenyl | CN | H | |
| 118 | $CH_3$ | Isopropenyl | Vinyl | H | 200-MHz-NMR-Spektrum in $CDCl_3$:<br>δ[ppm] = 2,02 (3H); 2,13 (breit, 1H); 2,33 (3H);<br>4,85 (1H); 5,19-5,5 (4H); 5,97-6,14 (1H);<br>7,07 (1H); 7,2 (1H); 7,4 (1H) |

Tabelle 4 (Fortsetzung)

| Beispiel | $R^1$ | $R^2$ | $R^3$ | X | Physikalische Eigenschaften |
|---|---|---|---|---|---|
| 119 | $CH_3$ | $C_2H_5$<br>\|<br>$-C=CH_2$ | H | H | |
| 120 | $CH_3$ | $CH_3$<br>\|<br>$-C=CH-CH_3$ | H | H | |
| 121 | $CH_3$ | $C_2H_5$<br>\|<br>$-C=CH-CH_3$ | H | H | $n_D^{22}$: 1,5325 |
| 122 | $CH_3$ | $CH_3$<br>\|<br>$-CH-CH_2-CH_3$ | H | H | 200-MHz-NMR-Spektrum in $CDCl_3$:<br>δ [ppm] = 0,88(3H); 1,22(3H); 1,64(2H); 1,86(1H); 2,33(3H); 3,0(1H); 4,72(2H); 7,22(3H) |
| 123 | $CH_3$ | $-CH(C_2H_5)_2$ | Isopropyl | H | |
| 124 | $C_2H_5$ | $CH_3$<br>\|<br>$-CH-CH_2-CH_3$ | H | H | |
| 125 | $CH_3$ | $CH_3$<br>\|<br>$-C=C(CH_3)_2$ | H | H | |
| 126 | $CH_3$ | $i-C_3H_7$<br>\|<br>$-C=CH_2$ | H | H | |
| 127 | $CH_3$ | 3,5-Diethyl-cyclohexyl | H | H | |

Tabelle 4 (Fortsetzung)

| Bei-spiel | $R^1$ | $R^2$ | $R^3$ | X | Physikalische Eigenschaften |
|---|---|---|---|---|---|
| 128 | $CH_3$ | $CH_2-CH_2-CH=CH_2$<br>\|<br>$-C=CH_2$ | H | H | |
| 129 | $CH_3$ | Vinyl | H | H | 300-MHz-NMR-Spektrum in $CDCl_3$:<br>δ [ppm] = 1,97(1H); 2,29(3H); 4,65(2H); 5,3(1H); 5,58(1H); 6,93-7,04(1H); 7,11-7,28(2H); 7,4(1H)<br>Schmp.: 38 - 43°C |
| 130 | $CH_3$ | $C_2H_5$<br>\|<br>$-C-(CH_3)_2$ | H | H | 300-MHz-NMR-Spektrum in $CDCl_3$:<br>δ [ppm]= 0,67(3H); 1,39(6H); 1,83(2H+1H); 2,46(3H); 4,66(2H); 7,09-7,31(3H) |

**Patentansprüche**

1. Benzonitrile, Benzaldehyde und Benzylalkohole der allgemeinen Formel I

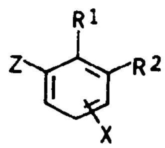

(I),

in der

$R^1$ Methyl oder Ethyl,

$R^2$ Alkyl, Alkenyl, Cycloalkyl, Cycloalkenyl, Bicycloalkyl, Bicycloalkenyl oder $C_1$-$C_5$-alkylsubstituiertes Cycloalkyl, Cycloalkenyl, Bicycloalkyl oder Bicycloalkenyl,

X Wasserstoff, Chlor oder Fluor und

Z die Gruppe -CN, CHO oder

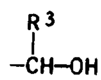

,

wobei $R^3$ für Wasserstoff, Cyano, $C_2$-$C_4$-Alkinyl, $C_2$-$C_4$-Alkenyl oder $C_1$-$C_4$-Alkyl steht,

bedeuten, mit der Maßgabe, daß $R^2$ nicht den Rest $-CH_2-CH=CH-B$ darstellt, wenn B für Wasserstoff, Alkyl oder Alkenyl steht und zugleich $R^1$ die Bedeutung Methyl und Z die Bedeutung

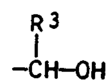

hat sowie mit der Maßgabe, daß $R^2$ nicht Methyl oder Ethyl bedeutet, wenn zugleich $R^1$ für Methyl und Z für

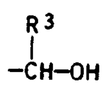

,

steht und ferner mit der Maßgabe, daß $R^2$ nicht Methyl bedeutet, wenn zugleich $R^1$ für Methyl oder Ethyl und Z für die Gruppe -CN oder -CHO steht.

2. Verbindungen nach Anspruch 1, dadurch gekennzeichnet, daß $R^1$ bis $R^3$ die in Anspruch 1 angegebene Bedeutung haben und X für Wasserstoff steht.

3. Verfahren zur Herstellung von Benzylalkoholen der allgemeinen Formel Ia

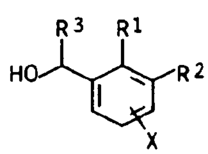

(Ia),

in der die Reste $R^1$ bis $R^3$ und X die in Anspruch 1 genannte Bedeutung haben, dadurch gekennzeichnet, daß man entsprechend substituierte Benzaldehyde Ib

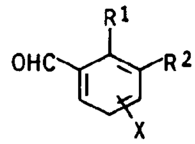

(Ib)

umsetzt mit

i) einem Reduktionsmittel im Fall von $R^3$=H,

ii) Blausäure oder einem Metallcyanid ggf. in Gegenwart einer Säure im Fall von $R^3$=CN

iii) Metallorganylen $MeR^3$ oder $R^3MeHal$ im Fall von $R^3$ = $C_2$-$C_4$-Alkinyl, $C_2$-$C_4$-Alkenyl oder $C_1$-$C_4$-Alkyl, wobei Me für ein Alkali-, Erdalkali- oder Übergangsmetall und Hal für Halogen steht.

**4.** Verfahren zur Herstellung von Benzaldehyden der allgemeinen Formel Ib

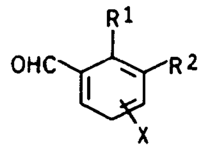

(Ib)

in der die Reste $R^1$, $R^2$ und X die in Anspruch 1 genannte Bedeutung haben, dadurch gekennzeichnet, daß man entsprechend substituierte Benzonitrile Ic

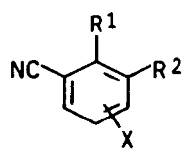

(Ic)

mit einem Reduktionsmittel zu Aldiminen II

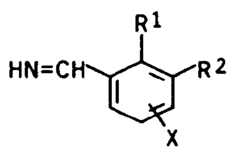

(II)

umsetzt und diese in an sich bekannter Weise verseift.

**5.** Verfahren zur Herstellung von Benzonitrilen der allgemeinen Formel Ic

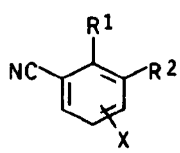

(Ic)

in der die Reste $R^1$, $R^2$ und X die in Anspruch 1 genannte Bedeutung haben, dadurch gekennzeichnet, daß man Verbindungen der allgemeinen Formel III

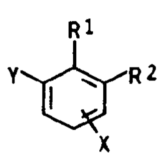

(III)

in der Y für Chlor oder Brom steht, mit Metallcyaniden in einem organischen Lösungsmittel umsetzt.

## Claims

1. A benzonitrile, benzaldehyde or benzyl alcohol of the formula I

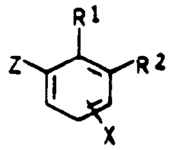

(I)

where $R^1$ is methyl or ethyl, $R^2$ is alkyl, alkenyl, cycloalkyl, cycloalkenyl, bicycloalkyl, bicycloalkenyl, or $C_1$-$C_5$-alkyl-substituted cycloalkyl, cycloalkenyl, bicycloalkyl or bicycloalkenyl, X is hydrogen, chlorine or fluorine and Z is -CN, CHO or

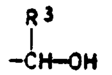 ,

where $R^3$ is hydrogen, cyano, $C_2$-$C_4$-alkynyl, $C_2$-$C_4$-alkenyl or $C_1$-$C_4$-alkyl, with the proviso that $R^2$ is not $-CH_2-CH=CH-B$ when B is hydrogen, alkyl or alkenyl and at the same time $R^1$ is methyl and Z is

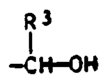

and with the proviso that $R^2$ is not methyl or ethyl when $R^1$ is methyl and at the same time Z is

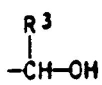 ,

and furthermore with the proviso that $R^2$ is not methyl when $R^1$ is methyl or ethyl and at the same time Z is -CN or -CHO.

2. A compound as claimed in claim 1, wherein $R^1$ to $R^3$ have the meanings stated in claim 1 and X is hydrogen.

3. A process for the preparation of a benzyl alcohol of the formula Ia

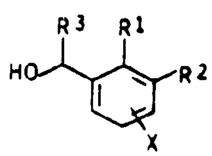

(Ia)

where $R^1$ to $R^3$ and X have the meanings stated in claim 1, wherein a correspondingly substituted benzaldehyde Ib

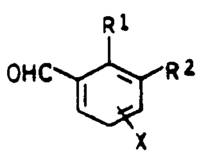

(Ib)

is reacted with
i) a reducing agent if $R^3$ is H,
ii) hydrocyanic acid or a metal cyanide in the presence or absence of an acid if $R^3$ is CN, or

iii) a metalorganyl $MeR^3$ or $R^3MeHal$ if $R^3$ is $C_2$-$C_4$-alkynyl, $C_2$-$C_4$-alkenyl or $C_1$-$C_4$-alkyl, Me being an alkali metal, alkaline earth metal or transition metal and Hal being halogen.

**4.** A process for the preparation of a benzaldehyde of the formula Ib

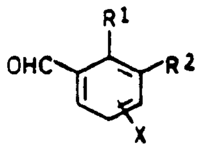

(Ib)

where $R^1$, $R^2$ and X have the meanings stated in claim 1, wherein a correspondingly substituted benzonitrile Ic

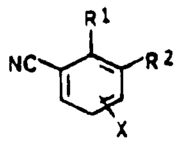

(Ic)

is reafted with a reducing agent to give an aldimine II

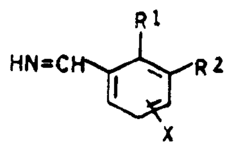

(II)

and hydrolyzing this in a conventional manner.

**5.** A process for the preparation of a benzonitrile of the formula Ic

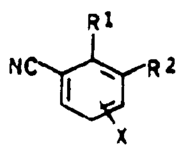

(Ic)

where $R^1$, $R^2$ and X have the meanings stated in claim 1, wherein a compound of the formula III

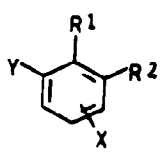

(III)

where Y is chlorine or bromine, is reacted with a metal cyanide in an organic solvent.

## Revendications

**1.** Benzonitriles, benzaldéhydes et alcools benzyliques de formule générale I

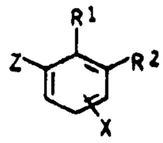

(I),

dans laquelle

$R^1$ représente un groupe méthyle ou éthyle,

$R^2$ représente un groupe alkyle, cycloalkyle, cycloalcényle, bicycloalkyle, bicycloalcényle ou un groupe cycloalkyle, cycloalcényle, bicycloalkyle ou bicycloalcényle portant des substituants alkyle en $C_1$-$C_5$,

X représente l'hydrogène, le chlore ou le fluor et

Z représente un groupe CN, CHO ou

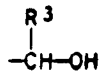 ,

dans lequel $R^3$ représente l'hydrogène, un groupe cyano, alcynyle en $C_2$-$C_4$, alcényle en $C_2$-$C_4$ ou alkyle en $C_1$-$C_4$,

sous réserve que $R^2$ ne peut représenter le groupe $-CH_2-CH=CH-B$ représente l'hydrogène, un groupe alkyle ou alcényle et que, simultanément, $R^1$ représente un groupe méthyle et Z le groupe

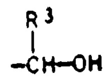

et sous réserve également que $R^2$ ne peut représenter un groupe méthyle ou éthyle lorsque, simultanément, $R^1$ représente un groupe méthyle et Z un groupe

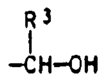 ,

et sous réserve également que $R^2$ ne peut représenter un groupe méthyle lorsque, simultanément, $R^1$ représente un groupe méthyle ou éthyle et Z un groupe -CN ou -CHO.

2. Composés selon la revendication 1, caractérisé en ce que les symboles $R^1$ à $R^3$ ont les significations indiquées dans la revendication 1, et X représente l'hydrogène.

3. Procédé de préparation d'alcools benzyliques de formule générale Ia

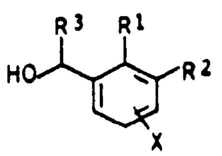

(Ia),

dans laquelle les symboles $R^1$ à $R^3$ et X ont les significations indiquées dans la revendication 1, caractérisé en ce que l'on fait réagir les benzaldéhydes substitués correspondants Ib

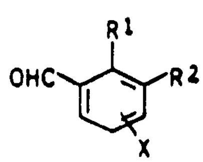

(Ib)

avec

i) un agent réducteur lorsque $R^3$ = H,

ii) l'acide cyanhydrique ou un cyanure métallique, éventuellement en présence d'un acide, lorsque $R^3$ = CN,

iii) des métaux-organyles $MeR^3$ ou $R^3MeHal$ dans les cas où $R^3$ = alcényle en $C_2$-$C_4$, alcényle en $C_2$-$C_4$ ou alkyle en $C_1$-$C_4$, Me représentant un métal alcalin, alcalino-terreux ou de transition et Hal un halogène.

4. Procédé de préparation des benzaldéhydes de formule générale Ib

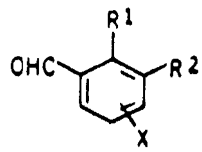

(Ib)

dans laquelle les symboles $R^1$, $R^2$ et X ont les significations indiquées dans la revendication 1, caractérisé en ce que l'on fait réagir les benzonitriles substitués correspondants Ic

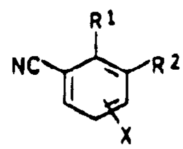

(Ic)

avec un agent réducteur, ce qui donne les aldimines II

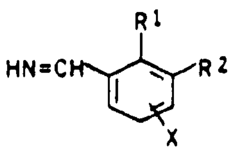

(II)

qu'on saponifie de manière connue en soi.

5. Procédé de préparation des benzonitriles de formule générale Ic

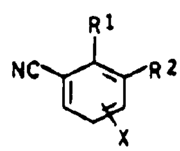

(Ic)

dans laquelle les symboles $R^1$, $R^2$ et X ont les significations indiquées dans la revendication 1, caractérisé en ce que l'on fait réagir des composés de formule générale III

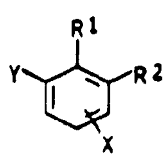

(III)

dans laquelle Y représente le chlore ou le brome, avec des cyanures métalliques dans un solvant organique.